(19) Europäisches Patentamt
European Patent Office
Office européen des brevets

(11) EP 2 697 192 B1

(12) **EUROPEAN PATENT SPECIFICATION**

(45) Date of publication and mention of the grant of the patent:
**21.10.2015 Bulletin 2015/43**

(21) Application number: **11869193.0**

(22) Date of filing: **11.08.2011**

(51) Int Cl.:
***C07C 239/20*** (2006.01) ***C07C 249/04*** (2006.01)
***C07C 249/12*** (2006.01) ***C07C 251/38*** (2006.01)

(86) International application number:
**PCT/CN2011/078296**

(87) International publication number:
**WO 2013/007054 (17.01.2013 Gazette 2013/03)**

(54) **METHOD FOR PREPARATION OF ALKOXY-AMINE HYDROCHLORIDE**

VERFAHREN ZUR HERSTELLUNG VON ALKOXY-AMIN-HYDROCHLORID

PROCÉDÉ POUR LA PRÉPARATION D'UN CHLORHYDRATE D'ALCOXYAMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2011 CN 201110191164**
**02.08.2011 CN 201110219604**

(43) Date of publication of application:
**19.02.2014 Bulletin 2014/08**

(73) Proprietors:
- **Ningbo Ocean Chemical New Materials Technology Co. Ltd.**
  **Zhejiang 315204 (CN)**
- **Ningbo Institute Of Technology Zhejiang University**
  **Ningbo, Zhejiang 315100 (CN)**

(72) Inventors:
- **ZHANG, Shengjian**
  **Ningbo**
  **Zhejiang 315100 (CN)**
- **LIN, Yong**
  **Zhejiang 315204 (CN)**

(74) Representative: **reuteler & cie SA**
**Chemin de la Vuarpillière 29**
**1260 Nyon (CH)**

(56) References cited:
**EP-A1- 1 548 000**
**WO-A1-00/55121**
**WO-A1-95/18788**
**CN-A- 1 660 785**
**CN-A- 101 503 375**
**CN-A- 101 648 887**
**CN-A- 102 093 253**
**US-A1- 2006 205 939**

- **ZHOU SHAODONG ET AL.: 'Green route to synthesize oxime' JOURNAL OF ZHEJIANG UNIVERSITY (ENGINEERING SCIENCE) vol. 44, no. 6, 2010, pages 1169 - 1172, XP008169821**
- **ZHANG SHENGJIAN ET AL.: 'O-alkylation of ketone oxime in ionic liquids' CHINESE JOURNAL OF ORGANIC CHEMISTRY vol. 30, no. 4, 2010, pages 606 - 610, XP008169855**

EP 2 697 192 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the Invention

The present invention relates to a method for preparation of alkoxy-amine hydrochloride.

Background of the Invention

Acetone oxime and butanone oxime have advantages such as low toxicity, environmental friendliness, and high reducibility, etc., and are excellent reducers, can be used to replace highly toxic $N_2H_4$ as deoxidizer and corrosion inhibitor for boilers, can be used as intermediates in organic synthesis and polyurethane sealing agents, and are raw materials for synthesis of medicines, pesticides, dyes, and organic silicon coupling agents.

Therefore, methods for preparation of acetone oxime and butanone oxime are disclosed in some patent publications, for example, in CN1556096A, a process for production of acetone oxime or butanone oxime by oxidation of acetone or butanone is disclosed, the process employs acetone or butanone, ammonia and hydrogen peroxide as raw materials, and comprising: dissolving acetone or butanone in isopropanol or tert-butyl alcohol at a proportion of 100-200g solvent/mol ketone; adding TS-1 catalyzer into the acetone or butanone solution at a proportion of 3-10g catalyzer/mol ketone and adding ammonia into the acetone or butanone solution at a proportion of 1.2-2 mol ammonia/mol ketone to prepare reaction solution; adding hydrogen peroxide by droplets at a proportion of 1.0-1.3 mol hydrogen peroxide/mol ketone for 0.25-12h, to obtain acetone oxime or butanone oxime product.

Though acetone oxime or butanone oxime can be obtained by using the method for preparation of acetone oxime or butanone oxime disclosed in CN1556096A, the method has the following disadvantages:

(1) In the method, the additional organic solvent (e.g., isopropanol or tert-butyl alcohol) has to be used, making the operation of separation of the acetone oxime or butanone oxime from the reaction product very dangerous; in addition, the organic solvent does not participate in the reaction but is only discharged as waste, thus increasing the burden of follow-up waste disposal.

(2) To increase the conversion ratio of the acetone or butanone, the molar ratio of the hydrogen peroxide to the ketone in the method is 1.0-1.3, that is to say, the amount of the hydrogen peroxide is equivalent to or more than the amount of the ketone. As a result, the hydrogen peroxide in the reaction system leaves some residue in the system after it participates in the oximation reaction of ketone and ammonia; the residual hydrogen peroxide not only accelerates the side reaction but also accelerates inactivation of the catalyzer, and thereby results in shortened useful life of the catalyzer, decreased conversion ratio of the acetone or butanone, and decreased selectivity of converting the acetone or the butanone to the acetone oxime or the butanone oxime.

Alkoxy-amine hydrochloride is also referred to O-substituted alkyl-hydroxylamine hydrochloride, in general formula as $H_2NOR_1 \cdot HCl$, wherein, $R_1$ may be $C_1$-$C_6$ alkyl, $C_2$-$C_6$ vinyl, $C_7$-$C_{13}$ aryl, or halogenated group of these groups (the halogen may be Br or Cl, etc.).

Alkoxy-amine hydrochloride is a kind of intermediate in organic synthesis, which is widely used and costly. The compound can be used as a reagent for amination of an alkoxy group, to introduce alkoxylamine groups into ketone compounds (particularly steroids) in organic synthesis and production of new medicines, used as branched chains of the medicine cefuroxime and the intermediate in production of herbicides such as traloxydim, clethodim and sethoxydim, etc., as well as intermediates in development of new medicines and new pesticides.

Existing methods for synthesis of alkoxylamine hydrochloride include: an alkoxylamine hydrochloride synthesis process introduced by Ji Yongxin (Chemistry and Adhesion, 2001, 5, p200-202), comprising: producing hydroxylamine sodium sulfite through the reaction of sodium bisulfite, sulfur dioxide and sodium nitrite, and producing the final product through alkylation, hydrolysis, neutralization and salification. However, the process requires liquid sulfur dioxide and sodium nitrite, which are highly toxic and highly pollutant; in addition, to obtain 1 ton of product, more than 10 tons of waste water that contains a large quantity of sodium sulfate, and a large quantity of waste gases such as nitrogen oxides, have to be produced, resulting in risks in safety and environmental protection.

A process for preparation of ethoxy amine through oximation reaction of ethyl acetate and hydroxylamine, protection of N, ethylization of diethyl sulfate into ether, and then hydrolysis is introduced by Li Wenxiao, et al (Fine Chemical Industrial Raw Materials & Intermediates, 2007, 22-23, p29). Wherein, impurities such as N-ethyl-O-ethyl amine have to be removed by reduced pressure distillation after ethylization, then, the intermediate has to be treated by acid hydrolysis, neutralization, and distillation to obtain ethoxy amine, at a yield of approx. 85%; however, the process have disadvantages, for example, the effect of impurity removal by distillation is unsatisfactory, and the process conditions of the ethylization and the like are difficult to control.

A method for preparation of O-substituted alkyl hydroxylamine hydrochloride (i.e., alkoxylamine hydrochloride)

is disclosed in US4981996A, comprising: producing O-substituted alkyl hydroxylamine hydrochloride directly through reaction of hydroxylamine-O-sulfonic acid and alkali metal alkoxide in polar solvent. However, that method requires dangerous fuming sulphuric acid, and the produced alkoxy amine has to be treated by reduced pressure distillation under certain pressure, and the operating conditions are difficult to control; in addition, with that method, a great deal of waste acid is produced, causing heavy burden on the follow-up waste disposal.

CN101503373 discloses a method of synthesizing methoxy amine hydrochloride but not the preparation of ketoxime compound and in the disclosed method the ketoxime ether (O-methyl-2-diacetylmonoxime ether) and hydrochloride acid are mixed and the mixed solution undergoes feeding and rectification at the middle of a rectifying column.

EP 1548000 discloses a process for producing an oxime (ketoxime compound) comprising: reacting a ketone, hydrogen peroxide and ammonia in the presence of crystalline titanosilicate, and the reaction is carried out using a solvent, wherein, the solvent is a mixture of alcohol and water.

WO 0055121 relates to a method for the continuous production of methoxyamine hydrochloride by dissociating acetone oxime methylether in presence of water and HCl, wherein the dissociation step is carried out in a reactor column of less than 20 theoretical dissociation steps and the amount of the removed overhead amounts to at least 30% of the supplied quantity. The acetone oxime methylether is introduced as a solution together with 31% strength hydrochloric acid.

ZHOU SHAODONG ET AL., 2010, JOURNAL OF ZHEJIANG UNIVERSITY (ENGINEERING SCIENCE), vol. 44(6), 1169-1172 relate to a green method to synthesize an oxime which uses no organic solvents. The reaction media is water and ketones react with ammonia and hydrogen peroxide under the catalysis of TS-1, producing oximes. It teaches towards the use of an amount of hydrogen peroxide higher than ketone.

To solve above technical problems, those skilled in the art tried to utilize the contact reaction between acetone oxime ether or butanone oxime ether and hydrochloric acid, and separate alkoxy-amine hydrochloride from the tower bottom via a distillation tower. However, the method is difficult to use in commercial process due to the following disadvantages:

(1) In the method, in order to separate acetone or butanone from alkoxy-amine hydrochloride in the mixture obtained through the reaction, an additional organic solvent (e.g., toluene, hexane, etc.) has to be added into the reaction system, making the operation of fractionation by distillation very dangerous;
(2) The reaction time is long, and there are many side reactions, thus resulting in a low yield of the alkoxy-amine hydrochloride;
(3) The method can only be used for batch production, thus resulting in a low production capacity.

Summary of the Invention

Such method comprises a step of preparing a ketoxime compound intermediate. The new method for preparation of ketoxime compound intermediate, overcomes the disadvantages in the method for preparation of acetone oxime or butanone oxime in the prior art, such as dangerous operation, short useful life of the catalyzer, low conversion ratio of the ketone compound, and low conversion ratio from the ketone compound to the ketoxime compound.

One object of the present invention is to provided a method for preparation of alkoxy-amine hydrochloride.

In the oximation reaction of ketone and ammonia, in order to obtain a high ketone conversion ratio, those skilled in the art usually use excessive amount of hydrogen peroxide as the oxidizer, as described in CN1556096A. In addition, using organic solvent as the reaction medium is usually deemed beneficial to the oximation reaction of ketone and ammonia, for example, in "Highly Selective Synthesis of Methyl Ethyl Ketone Oxime through Ammoximation over Ti-MWW" (Applied Catalysis A: General 327 (2007) 22-31, Liu Yueming, et al), an instruction is provided, which specifies that water is not preferred as the solvent for the oximation reaction of ketone and ammonia if TS-1 catalyzer is used because the conversion ratio of the butanone is low. However, the inventors of the present invention found: by reducing the amount of hydrogen peroxide to be lower than its stoichiometry required in the oximation reaction of ketone and ammonia, and utilizing water as the solvent, the yield and purity of the ketoxime compound can be increased significantly, the byproducts can be reduced greatly, and thereby the useful life of the catalyzer can be prolonged greatly, though the conversion ratio of the ketone is reduced to some degree; moreover, excessive ketone can be directly recovered and reused; thus, after cyclic reaction, the conversion ratio of ketone is still high.

In addition, the inventors of the present invention also found: in the process of preparation of the alkoxy-amine hydrochloride, by allowing the reaction of the ketoxime ether compound and the hydrochloric acid to be performed in a catalytic distillation tower, not only the additional organic solvent can be omitted, but also the target product can be obtained at high purity and high yield, the procedure of the separation can be eliminated, and thereby the production cost can be reduced, due to the integration of the reaction and separation.

The present invention further provides a method for preparation of alkoxy-amine hydrochloride, comprising the following steps:

(1) feeding a ketone compound, hydrogen peroxide, water, and ammonia into a reactor for contact reaction under the conditions of oximation reaction of ketone and ammonia, in the presence of a titanium-silicon molecular sieve catalyst to prepare ketoxime compound, wherein, the molar ratio of the hydrogen peroxide 5 to the ketone compound is 0.1 or more and less than 1
(2) allowing the ketoxime compound obtained in step (1) to have contact reaction with a halohydrocarbon under the conditions of substitution reaction, and separating ketoxime ether compound from the product of the contact reaction;
(3) feeding the ketoxime ether compound obtained in step (2) and hydrochloric acid solution respectively into a second rectification tower continuously for catalytic distillation, and separating alkoxy-amine hydrochloride from the liquid at the tower bottom of the second rectification tower wherein the process for feeding the ketoxime ether compound obtained in step (2) and the hydrochloric acid solution respectively into the second rectification tower continuously comprises: feeding the ketoxime ether compound obtained in step (2) into the second rectification tower through a first feed inlet, and feeding the hydrochloric acid solution into the second rectification tower through a second feed inlet, wherein, the number of plates or the theoretical plate number D1 from the first feed inlet to the tower bottom and the number of plates or the theoretical plate number D2 from the second feed inlet to the tower bottom is 30-80% of the total number of plates or the theoretical plate number of the second rectification tower respectively, and D1<D2.

In the step for preparation of ketoxime compound intermediate provided in method of the present invention, no additional organic solvent is required, and therefore the separation process of the ketoxime compound is safer; moreover, since the molar ratio of the hydrogen peroxide to the ketone compound is lower than 1, the side reactions can be reduced, and the useful life of the titanium-silicon molecular sieve catalyst is longer.
With the method for preparation of alkoxy-amine hydrochloride provided in the present invention, since the process is performed in a catalytic distillation tower, no additional organic solvent is required, and the target product can be obtained at high purity and high yield, and the separation procedure can be omitted, and thereby the purpose of reduction of production cost can be attained, due to the integration of the reaction and separation.

Detailed Description of the Embodiments

Preferably, step (1) for preparation of ketoxime compound intermediate provided in the present method of the invention may further comprise: separating the titanium-silicon molecular sieve catalyst after the contact reaction and the mixed liquid A that contains ketoxime compound, the ketone compound and water from the product of the contact reaction, separating the ketoxime compound and the mixed liquid B that contains the ketone compound and water from the mixed liquid A, and then feeding the mixed liquid B as a part of input materials of the reactor back to the reactor. In this case, the ketone compound that didn't react completely in the reactor can be used cyclically.
In step (1) for preparation of ketoxime compound intermediate provided in the present invention, although the conversion ratio of ketone compound in a single cycle of the contact reaction is decreased to some degree because no additional organic solvent is used, the ketone compound can be converted completely after cyclic contact reactions, since the ketone compound that does not react completely can be recovered and used cyclically. Therefore, with the method provided in the present description, the ketone compound can be still obtained at a high conversion ratio even though no additional organic solvent is used. Moreover, without additional organic solvent, not only the risk and complexity of the separation process of the ketoxime compound, but also the resultant waste can be greatly reduced. Therefore, step (1) for preparation of ketoxime compound intermediate provided in the present method of the invention has high economic benefit and is suitable for commercial production.
In step (1), the ketone compound may be an ordinary monoketone compound, preferably a $C_3$-$C_6$ monoketone compound, particularly acetone or butanone.
In step (1) for preparation of ketoxime compound intermediate provided in the present method of the invention, the process of separation of the ketoxime compound and the mixed liquid B that contains the ketone compound and water from the mixed liquid A can be implemented by means of any ordinary separation process. Preferably, the process of separation of the ketoxime compound and the mixed liquid B that contains the ketone compound and water from the mixed liquid A is implemented in a first rectification tower, and the mixed liquid B that contains the ketone compound and water is collected from the tower top of the first rectification tower, and the mixture that contains the ketoxime compound is collected from the tower bottom of the first rectification tower. In addition, such a separation manner is especially suitable for the case in which the ketone compound is acetone or butanone. More preferably, the preferred operating conditions of the first rectification tower include: the theoretical plate number is 20-80, more preferably 30-60; the temperature at the tower bottom is 80-130°C, more preferably 100-120°C; the reflux ratio is not lower than 2:1, more preferably 5-10:1.
The first rectification tower is filled with filler, which may be any ordinary filler for rectification tower, for example, the filler may be at least one of metal mesh, ceramics and polytetrafluoro-material (e.g., polytetrafluoroethene).

To collect the ketoxime compound product at a high purity, step (1) provided in the present invention may further comprise: extracting the mixture that contains the ketoxime compound for 3-5 times with halohydrocarbon, collecting the extract, and treating the extract by distillation at normal pressure or reduced pressure at a temperature not higher than 70°C, preferably a temperature in the range of room temperature to 70°C, and then distilling to remove the solvent and thus obtain the ketoxime compound product

Preferably, step (1) for preparation of ketoxime compound provided in the present invention may further comprise: recycling and reusing the separated titanium-silicon molecular sieve catalyst after the contact reaction as at least a part of the titanium-silicon molecular sieve catalyst for the contact reaction. In this case, the separated titanium-silicon molecular sieve catalyst after the contact reaction can be recovered and reused, and thereby the utilization ratio of the titanium-silicon molecular sieve catalyst can be increased, and the production cost can be reduced.

In a preferred embodiment of the present invention, the process of separation of the titanium-silicon molecular sieve catalyst after the contact reaction and the mixed liquid A from the product of the contact reaction can be implemented by means of any ordinary separation process, preferably implemented in a membrane filter. In this case, since the membrane filter can filter off tiny solid particles, in the step for preparation of ketoxime compound provided in the present invention, unshaped titanium-silicon molecular sieve catalyst powder can be used as the catalyzer. In addition, since the unshaped titanium-silicon molecular sieve catalyst powder has higher catalytic activity than the shaped titanium-silicon molecular sieve catalyst, and thus it is more beneficial to increase the conversion ratio of the ketone compound.

In the present invention, the membrane filter can be any ordinary membrane filter, such as a ceramic membrane filter. The bore diameter of the interior of the membrane filter is smaller than the particle diameter of the catalyzer, and is preferably 0.03-0.4μm.

The step for preparation of ketoxime compound in the method provided in the present invention, the oximation reaction of ketone and ammonia can be expressed with the following formula (1):

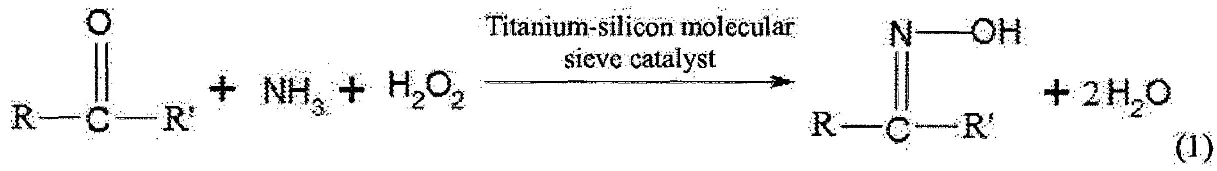


(1)

It can be seen from the above formula: the theoretical molar ratio of the hydrogen peroxide to the ketone compound in the reaction is 1.

Although a high conversion ratio of the ketone compound can be obtained in individual cycles of the contact reaction when the molar ratio of the hydrogen peroxide to the ketone compound is more than or equal to I in the input materials, the residual hydrogen peroxide will be left in the reaction system when the amount of the hydrogen peroxide is equivalent to or more than the amount of the ketone compound, and the residual hydrogen peroxide will not only accelerate the side reactions but also accelerate inactivation of the catalyzer, resulting in remarkably shortened useful life of the catalyzer and remarkably decreased selectivity for converting the ketone compound to the ketoxime compound (i.e., the selectivity of the ketoxime compound). However, the inventors of the present invention found: in the process of the oximation reaction of ketone and ammonia, the above disadvantages can be overcame when the molar ratio of the hydrogen peroxide to the ketone compound in the input materials is 0.1 or more and less than 1, and thereby the useful life of the catalyzer can be prolonged, and a higher selectivity of the ketoxime compound can be obtained.

In a preferred embodiment, in order to improve the reaction efficiency and further prolong the useful life of the catalyzer, the molar ratio of the hydrogen peroxide to the ketone compound is preferably 0.5-0.95, more preferably 0.7-0.9.

In the present invention, the hydrogen peroxide is usually added in the form of hydrogen peroxide solution, and the concentration of the hydrogen peroxide solution may be 28-50 wt.%, preferably 28-30 wt.%.

In the step for preparation of ketoxime compound provided in the present invention, there is not special restriction to the conditions of the oximation reaction of ketone and ammonia, that is to say, all ordinary conditions of the oximation reaction of ketone and ammonia are applicable to the present invention. In a preferred embodiment, the conditions of the oximation reaction of ketone and ammonia may include: the reaction temperature is 45-85°C, preferably 50-80°C; the reaction time is 0.5-6h, preferably 1-5h.

In the step for preparation of ketoxime compound provided in the present invention, the titanium-silicon molecular sieve catalyst can be fed in an amount equivalent to the feeding amount of any ordinary catalyzer. However, in order to ensure the catalyzer has enough catalytic activity in the reaction system and reduce the feeding amount of the titanium-silicon molecular sieve catalyst as far as possible so as to reduce the production cost, the weight ratio of the titanium-silicon molecular sieve catalyst to the ketone compound is preferably 0.03-0.15:1, more preferably 0.05-0.1:1.

In the step for preparation of ketoxime compound provided in the present invention, the weight ratio of the water to the ketone compound is preferably 0.5-6:1. When the water and the ketone compound are fed at the preferred weight

ratio, the titanium-silicon molecular sieve catalyst in the reaction system will not be inactivated too quickly due to lack of water and the reaction efficiency will not be decreased due to excessive water. In a more preferred embodiment, the weight ratio of the water to the ketone compound is 1-4:1.

In the step for preparation of ketoxime compound provided in the present invention, the water in the reaction system mainly comprises water added in the form of hydrogen peroxide solution, water added separately, and water added selectively in the form of ammonia water. In the method for preparation of ketoxime compound provided in the present invention, there is not special restriction to the feeding amount of the ammonia, that is to say, the ammonia can be fed in an amount equivalent to the feeding amount of ammonia for the ordinary oximation reaction of ketone and ammonia. Preferably, the molar ratio of the ammonia to the ketone compound is 1.4-2:1. In the preferred embodiment, the reactivity of the reaction system will not be decreased due to lack of ammonia, and the alkalinity of the reaction system will not be too high and cause accelerated inactivation of the catalyzer due to excessive ammonia. In a more preferred embodiment, the molar ratio of the ammonia to the ketone compound is 1.5-1.7:1.

In the present invention, the ammonia can be added in the form of ammonia gas or ammonia water, and the concentration of the ammonia water may be 20-50 wt.%, preferably, the ammonia is added in the form of ammonia gas.

In the present invention, in order to obtain the ketoxime compound at a high yield, the titanium-silicon molecular sieve catalyst is preferably a titanium-silicon molecular sieve catalyst with a hollow structure. The radial length of the cavity part of the hollow structure of the titanium-silicon molecular sieve catalyst is 5-300nm, the amount of the absorbed benzene on the titanium-silicon molecular sieve catalyst measured under the conditions of 25°C, $P/P_0$=0.10 and 1h adsorption time is at least 70mg/g, and there is a hysteresis loop between the low-temperature nitrogen adsorption isotherm line and the desorption isotherm line of the titanium-silicon molecular sieve catalyst. The titanium-silicon molecular sieve catalyst may be the titanium-silicon molecular sieve itself or any preformed catalyst of the titanium-silicon molecular sieve. The titanium-silicon molecular sieve catalyst with a hollow structure is available commercially (e.g., commercial TS-1 molecular sieve catalyst) or can be prepared through an ordinary process, and its preparation process can refer to the process disclosed in CN1301599A, particularly the examples 1-11 therein.

In the step for preparation of ketoxime compound provided in the present invention, the reactor may be any ordinary reactor, for example, it may be a slurry bed reactor.

In the step for preparation of ketoxime compound intermediate provided in the present invention, there is no special restriction to the feeding sequence of acetone, water, ammonia, hydrogen peroxide, and titanium-silicon molecular sieve catalyst, as long as they are mixed at appropriate proportions to contact and react with each other under the conditions of the oximation reaction of ketone and ammonia, so as to attain the objects of the present invention.

In step (2), there is no special restriction to the conditions of the substitution reaction, that is to say, all conditions for ordinary substitution reaction that can ensure the ketoxime compound and halohydrocarbon to have substitution reaction are applicable to the present invention. Preferably, the conditions of the substitution reaction may include: the reaction temperature is 20-70 °C, and the reaction time is 1-3h.

In step (2), there is no special restriction to the feeding amount of the ketoxime compound and the halohydrocarbon, that is to say, the feeding amount of the ketoxime compound and the halohydrocarbon can be chosen appropriately within the ordinary range of feeding amount. The molar ratio of the halohydrocarbon to the ketoxime compound is preferably 1-1.3:1, more preferably 1.05-1.2:1.

In step (2), there is no special restriction to the category of the halohydrocarbon, that is to say, the halohydrocarbon may be any ordinary halohydrocarbon, compound, for example, the general formula of the halohydrocarbon maybe $R_1X_t$, where, $R_1$ is $C_1$-$C_6$ alkyl, $C_2$-$C_6$ vinyl, $C_7$-$C_{13}$ aryl, or halogenated group of these groups, X is chlorine or bromine, t is the number of the X atom, and t is 1. Preferably, the halohydrocarbon is selected from methyl chloride, ethyl chloride, 1-chloropropylene, or benzyl chloride.

In step (2), the contact reaction between the ketoxime compound and the halohydrocarbon is preferably performed in the presence of an alkali metal hydroxides and a solvent, and may be expressed with the following formula (2).

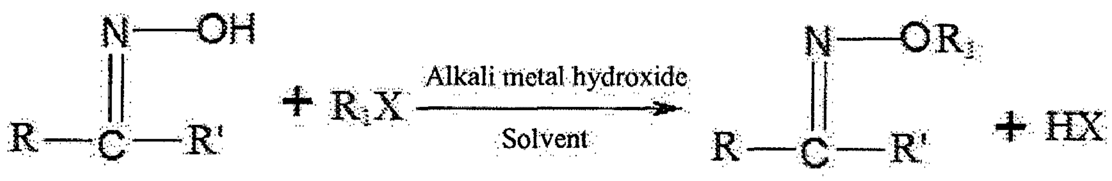


(2)

The weight ratio of the solvent to the ketoxime compound may be 1-20:1, preferably 3-20:1. The molar ratio of the alkali metal hydroxides to the ketoxime compound may be 1-1.5:1, preferably 1.1-1.3:1.

In the present invention, the alkali metal catalyst may be any ordinary water-soluble alkali metal hydroxide, for example, it may be selected from sodium hydroxide and/or potassium hydroxide.

In the present invention, the solvent in step (2) may be any ordinary organic solvent that is used in the process of preparation of the ketoxime ether compound from the ketoxime compound and the halohydrocarbon, for example, it may be at least one of selected from dimethyl sulfoxide, sulfolane, [BMIM]Cl ion liquid, and [BMIM]OH ion liquid.

In step (2), the separation of ketoxime ether compound from the reaction product may be implemented with any ordinary method, for example, the method may comprise: cooling down the reaction product to room temperature, adding water, extracting with extraction solvent such as ether, cyclohexane or hexane, and then rectifying and separating the extract, and distilling to remove the solvent and thus obtain the ketoxime ether product.

In the method for preparation of alkoxy-amine hydrochloride provided in the present invention, in step (3), the reaction between the hydrochloric acid solution and the ketoxime ether compound may be expressed in the following formula (3).

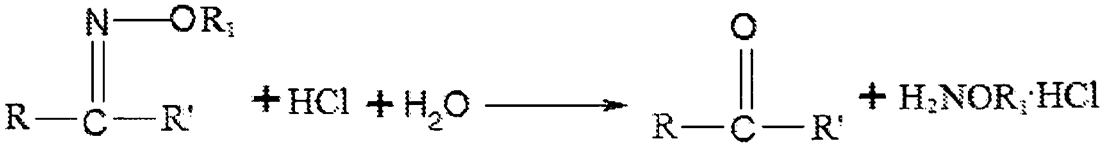

(3)

In a preferred embodiment of the present invention, in step (3), the method for feeding the ketoxime ether compound obtained in step (2) and the hydrochloric acid solution respectively into a second rectification tower continuously comprises: feeding the ketoxime ether compound obtained in step (2) into the second rectification tower through a first feed inlet, and feeding the hydrochloric acid solution into the second rectification tower through a second feed inlet, wherein, the number of plates or the theoretical plate number D1 from the first feed inlet to the tower bottom and the number of plates or the theoretical plate number D2 from the second feed inlet to the tower bottom is 30-80%, preferably 40-60% of the total number of plates or the theoretical plate number of the second rectification tower respectively, and D1<D2, that is to say, the second feed inlet is above the first feed inlet.

In the above preferred embodiment, by feeding the hydrochloric acid solution and the ketoxime ether compound respectively into the second rectification tower at the middle part of the second rectification tower and arranging the feed inlet of the hydrochloric acid solution above the feed inlet of the ketoxime ether compound, the ketone compound produced from the reaction between the hydrochloric acid and the ketoxime ether compound may be discharged from the tower top of the second rectification tower, without any additional organic solvent (e.g., toluene, hexane, etc.); in addition, in this preferred embodiment, the hydrochloric acid solution can contact with the ketoxime ether compound in counter-flow, and thereby the reaction rate can be increased, the reaction time can be reduced, and the side reactions can be reduced; moreover. Furthermore, according to this preferred embodiment, continuous production can be implemented, and the yield of the entire process can be improved.

In step (3), the reaction and rectification happen at the same time, that is to say, while the hydrochloric acid solution and the ketoxime ether compound are fed into the second rectification tower at the middle part of the second rectification tower respectively and react with each other, the product from the reaction is separated at the middle part of the second rectification tower; as a result, the heavy constituent, i.e. alkoxy-amine hydrochloride, falls into the tower caldron, while the light constituent, i.e. ketone compound, is discharged from the tower top.

More preferably, the difference between D1 and D2 is 5-30%, more preferably 5-20% of the total number of plates or the theoretical plate number of the second rectification tower.

In step (3), in order to increase the yield of the alkoxy-amine hydrochloride, the total number of plates or the theoretical plate number of the second rectification tower can be 20-100, preferably 30-80.

In step (3), the temperature at the tower bottom of the second rectification tower may be 70-110°C, preferably 85-95°C; the temperature at the tower top may be 50-65 °C, preferably 55-60°C.

It can be seen from the formula (3): the distillate from the tower top of the second rectification tower is mainly mixed liquid of ketone, water and unreacted HCl; the product of the tower caldron mainly comprises alkoxy-amine hydrochloride, water, and unreacted ketoxime ether compound, and the content of the alkoxy-amine hydrochloride is usually as high as 50-60 wt.%.

The molar ratio of HCl in the hydrochloric acid solution to the ketoxime compound in step (2) maybe 1.1-2.5:1, preferably 1.5-2:1. The concentration of the hydrochloric acid solution may be 15-35 wt.%, preferably 20-25 wt%.

It can be seen from the formula (3): another product in step (3) is the same as the ketone compound in step (1), and the ketone compound is discharged from the tower top of the second rectification tower in the catalyzed rectification process. In addition, since no additional organic solvent is added in step (3), the ketone compound in the distillate of the tower top may be directly recovered and reused in step (1), with or without being treated by concentration. Moreover, the presence of some HCl in the distillate of the tower top is beneficial to the stabilization of the hydrogen peroxide in step (1). Therefore, in another preferred embodiment of the present invention, the method further comprises: recycling

and reusing the distillate of the tower top of the second rectification tower as at least a part of the ketone compound in step (1) directly or after treatment by concentration. In this preferred embodiment, the ketone compound produced from the reaction between the hydrochloric acid solution and the ketoxime ether compound can be recovered and reused, thus solving the problem of the destination of the produced ketone compound on one hand and solving the problem of the source of the ketone compound in step (1) on the other hand, and thereby reducing the production cost.

In step (3), the separation of alkoxy-amine hydrochloride from the liquid on the tower bottom of the second rectification tower may be implemented with any ordinary separation method, for example, the separation method may comprises: distilling the liquid on the tower bottom of the second rectification tower at a reduced pressure, until solid is separated out, cooling the liquid to the room temperature and then filtering the liquid, washing the solid obtained by filtration with lower alcohol (e.g., methanol) and then drying.

In the present invention, the second rectification tower is filled with filler, which may be any ordinary filler for rectification towers, for example, the filler may be ceramics and/or polytetrafluoro-material (e.g., polytetrafluoroethene).

According to a particular aspect, is provided a method for preparation of alkoxy-amine hydrochloride, comprising the following steps:

(1) feeding a ketone compound, hydrogen peroxide, water, and ammonia into a reactor for contact reaction under the conditions of oximation reaction of ketone and ammonia, in the presence of a titanium-silicon molecular sieve catalyst, to prepare ketoxime compound, wherein, the molar ratio of the hydrogen peroxide to the ketone compound is 0.1 or more and less than 1;

(2) allowing the ketoxime compound obtained in step (1) to have contact reaction with a halohydrocarbon under the conditions of substitution reaction, and separating ketoxime ether compound from the product of the contact reaction;

(3) feeding the ketoxime ether compound obtained in step (2) and hydrochloric acid solution respectively into a second rectification tower continuously for catalytic distillation, and separating alkoxy-amine hydrochloride from the liquid at the tower bottom of the second rectification tower, wherein the process for feeding the ketoxime ether compound obtained in step (2) and the hydrochloric acid solution respectively into the second rectification tower continuously comprises: feeding the ketoxime ether compound obtained in step (2) into the second rectification tower through a first feed inlet, and feeding the hydrochloric acid solution into the second rectification tower through a second feed inlet, wherein, the number of plates or the theoretical plate number D1 from the first feed inlet to the tower bottom and the number of plates or the theoretical plate number D2 from the second feed inlet to the tower bottom is 30-80% of the total number of plates or the theoretical plate number of the second rectification tower respectively, and $D1<D2$, wherein in step (1), the molar ratio of the hydrogen peroxide to the ketone compound is 0.5-0.95, preferably 0.7-0.9; the conditions of oximation reaction of ketone and ammonia include: the reaction temperature is 45-85°C, preferably 50-80°C, the reaction time is 0.5-6h, preferably 1-5h; the weight ratio of the titanium-silicon molecular sieve catalyst to the ketone compound is 0.03-0.15:1, preferably 0.05-0.1:1; the weight ratio of the water to the ketone compound is 0.5-6:1, preferably 1-4:1; the molar ratio of the ammonia to the ketone compound is 1-1.8:1, preferably 1.5-1.7:1.

According to another particular aspect, is provided a method for preparation of alkoxy-amine hydrochloride wherein, in step (1), the method further comprising: separating the titanium-silicon molecular sieve catalyst after the contact reaction and the mixed liquid A that contains ketoxime compound, the ketone compound and water from the product of the contact reaction, separating the ketoxime compound and the mixed liquid B that contains the ketone compound and water from the mixed liquid A, and then feeding the mixed liquid B as a part of input materials of the reactor back to the reactor.

According to another further particular aspect, is provided a method for preparation of alkoxy-amine hydrochloride wherein, in step (1), the method further comprising: separating the titanium-silicon molecular sieve catalyst after the contact reaction and the mixed liquid A that contains ketoxime compound, the ketone compound and water from the product of the contact reaction, separating the ketoxime compound and the mixed liquid B that contains the ketone compound and water from the mixed liquid A, and then feeding the mixed liquid B as a part of input materials of the reactor back to the reactor and wherein, in step (1), the process for separating the ketoxime compound and the mixed liquid B that contains the ketone compound and water from the mixed liquid A is performed in a first rectification tower, and wherein, the theoretical plate number of the first rectification tower is 20-80, preferably 30-60; the temperature at the tower bottom is 80-130°C, preferably 100-120°C; and the reflux ratio is not lower than 2:1, preferably 5-10:1. According to another further particular aspect, is provided a method for preparation of alkoxy-amine hydrochloride wherein, in step (1), the method further comprising: separating the titanium-silicon molecular sieve catalyst after the contact reaction and the mixed liquid A that contains ketoxime compound, the ketone compound and water from the product of the contact reaction, separating the ketoxime compound and the mixed liquid B that contains the ketone compound and water from the mixed liquid A, and then feeding the mixed liquid B as a part of input materials of the reactor back to the reactor and the method further comprising: recycling and reusing the separated titanium-silicon molecular sieve catalyst after the

contact reaction as at least a part of the titanium-silicon molecular sieve catalyst for the contact reaction.

According to another further particular aspect, is provided a method for preparation of alkoxy-amine hydrochloride wherein, in step (1), the method further comprising: separating the titanium-silicon molecular sieve catalyst after the contact reaction and the mixed liquid A that contains ketoxime compound, the ketone compound and water from the product of the contact reaction, separating the ketoxime compound and the mixed liquid B that contains the ketone compound and water from the mixed liquid A, and then feeding the mixed liquid B as a part of input materials of the reactor back to the reactor and the process for separating of the titanium-silicon molecular sieve catalyst after the contact reaction and the mixed liquid A from the product of the contact reaction is implemented in a membrane filter.

According to another further particular aspect, is provided a method of the invention wherein the ketone compound is C3-C6 monoketone, preferably acetone or butanone; the titanium-silicon molecular sieve catalyst is a titanium-silicon molecular sieve catalyst with a hollow structure.

Hereunder the present invention will be further detailed by the following examples.

In the following examples and comparative examples, the utilization ratio of the hydrogen peroxide, the selectivity of the acetone oxime and the yield of the alkoxy-amine hydrochloride are obtained by calculation with the following formulae:

Utilization ratio of the hydrogen peroxide = mole number of the produced acetone oxime / mole number of the added hydrogen peroxide × 100%

Selectivity of the acetone oxime = mole number of the produced acetone oxime / mole number of the converted acetone × 100%

Yield of the alkoxy-amine hydrochloride = mole number of the produced alkoxy-amine hydrochloride / mole number of the inputted ketoxime ether compound × 100%

Example 1

This example is provided to describe the method for preparation of ketoxime compound intermediate provided in the method of the present invention.

Feed acetone, hydrogen peroxide solution at 27.5 wt.% concentration, ammonia gas, water, and TS-1 molecular sieve catalyst (prepared by using the method disclosed in example 1 in CN1301599A) continuously into a 5L slurry bed reactor equipped with a ceramic membrane separator, the weight ratio of the amount of the acetone : the water : the TS-1 molecular sieve catalyst is 1:2:0.05, the molar ratio of the ammonia gas to the acetone is 1.6:1, and the molar ratio of hydrogen peroxide added in the form of hydrogen peroxide solution to the acetone is 0.9:1, and then allow the raw materials of reaction to react with each other for 1h at 50°C by controlling the input flow and output flow of the slurry bed reactor. Meanwhile, separate the reacted materials discharged from the slurry bed reactor into reacted TS-1 molecular sieve catalyst and reacted liquid via the ceramics membrane separator, and return the reacted TS-1 molecular sieve catalyst to the slurry bed reactor as the raw TS-1 molecular sieve catalyst by back-flushing; feed the reacted liquid into a first rectification tower for rectification and separation, wherein, the theoretical plate number of the first rectification tower is 30, the filler in the first rectification tower is stainless steel mesh, the diameter of the tower is 600mm, and the height of the tower is 12m; collect aqueous acetone that contains about 55 wt.% acetone from the tower top, and return the aqueous acetone as raw acetone to the slurry bed reactor; while cool down the liquid of the tower bottom to room temperature, and extract for 3 times with tetrachloroethylene, combine the extract, and then distill the combined extract at a reduced pressure at about 65°C to remove the tetrachloroethylene and obtain acetone oxime product; the melting point of the acetone oxime product is 58.2-60.1°C, and the Infrared Spectrum and the Mass Spectrum data of the acetone oxime product is as follows:

IR, $\nu_{max}$ / $cm^{-1}$: 3200, 2920, 2896, 1681, 1498, 1371, 1268, 1072, 949, 81;
MS *m*/*z*(%): 73 ($M^+$, 100), 58 (64), 54 (21), 42 (21), 41 (21), 31 (31), 28 (42), 15 (45).

Allow the above process to proceed for 200h continuously, and calculate the utilization ratio of the hydrogen peroxide and the selectivity of the acetone oxime at different times, according to the feeding amount and the residual amount of the acetone and the yield of the acetone oxime. The result is shown in the following Table 1.

Comparative example 1

Prepare acetone oxime as the method described in example 1, with the following difference: the water (water added in the form of pure water, excluding water in the hydrogen peroxide solution) is replaced with tert-butyl alcohol in the same weight, and the molar ratio of hydrogen peroxide added in the form of hydrogen peroxide solution to the acetone is 1.1:1.

Allow the above process to proceed for 150h continuously, and at that point, the catalyzer is inactivated severely, then stop the experiment. Calculate the utilization ratio of the hydrogen peroxide and the selectivity of the acetone oxime at different times, according to the feeding amount and the residual amount of the acetone and the yield of the acetone oxime. The result is shown in the following Table 1.

Example 2

This example is provided to describe the method for preparation of ketoxime compound intermediate provided in the method of the present invention.

Feed acetone, hydrogen peroxide solution at 30 wt.% concentration, ammonia gas, water, and TS-1 molecular sieve catalyst (prepared by using the method disclosed in example 2 in CN1301599A) continuously into a 5L slurry bed reactor equipped with a ceramic membrane separator, the weight ratio of the amount of the acetone : the water: the TS-1 molecular sieve catalyst is 1:1:0.1, the molar ratio of the ammonia gas to the acetone is 1.5:1, and the molar ratio of hydrogen peroxide added in the form of hydrogen peroxide solution to the acetone is 0.8:1, and then allow the raw materials of reaction to react with each other for 0.5h at 65°C by controlling the input flow and output flow of the slurry bed reactor. Meanwhile, separate the reacted materials discharged from the slurry bed reactor into reacted TS-1 molecular sieve catalyst and reacted liquid via the ceramics membrane separator, and return the reacted TS-1 molecular sieve catalyst to the slurry bed reactor as raw TS-1 molecular sieve catalyst to the slurry bed reactor by back-flushing; feed the reacted liquid into the a first rectification tower for rectification and separation, wherein, the theoretical plate number of the first rectification tower is 30, the filler in the first rectification tower is stainless steel mesh, the diameter of the tower is 600mm, and the height of the tower is 12m; collect aqueous acetone that contains about 55 wt.% acetone from the tower top, and return the aqueous acetone as raw acetone to the slurry bed reactor; while cool down the liquid of the tower bottom to room temperature, and extract for 3 times with tetrachloroethylene, combine the extract and then distill the combined extract at a reduced pressure at about 65 °C to remove the tetrachloroethylene and obtain acetone oxime product; the melting point of the acetone oxime product is 58.4-60.0°C, and the Infrared Spectrum and the Mass Spectrum data of the acetone oxime product is as follows:

IR, $\nu_{max}$ / $cm^{-1}$: 3201, 2920, 2898, 1681, 1500, 1371, 1267, 1072, 949, 81;
MS *m*/*z*(%)*:* 73 ($M^+$, 100), 58 (64), 54 (21), 42 (21), 41 (21), 31 (31), 28 (42), 15 (45).

Allow the above process to proceed for 200h continuously, and calculate the utilization ratio of the hydrogen peroxide and the selectivity of the acetone oxime at different times, according to the feeding amount and the residual amount of the acetone and the yield of the acetone oxime. The result is shown in the following Table 1.

Example 3

This example is provided to describe the method for preparation of ketoxime compound intermediate provided in the method of the present invention.

Feed acetone, hydrogen peroxide solution at 30 wt.% concentration, ammonia gas, water, and TS-1 molecular sieve catalyst (prepared by using the method disclosed in example 3 in CN1301599A) continuously into a 5L slurry bed reactor equipped with a ceramic membrane separator, the weight ratio of the amount of the acetone : the water: the TS-1 molecular sieve catalyst is 1:3:0.08, the molar ratio of the ammonia gas to the acetone is 1.7:1, and the molar ratio of hydrogen peroxide added in the form of hydrogen peroxide solution to the acetone is 0.7:1, and then allow the raw materials of reaction to react with each other for 0.5h at 80°C by controlling the input flow and output flow of the slurry bed reactor. Meanwhile, separate the reacted materials discharged from the slurry bed reactor into reacted TS-1 molecular sieve catalyst and reacted liquid via the ceramics membrane separator, and return the reacted TS-1 molecular sieve catalyst to the slurry bed reactor as raw TS-1 molecular sieve catalyst to the slurry bed reactor by back-flushing; feed the reacted liquid into the a first rectification tower for rectification and separation, wherein, the theoretical plate number of the first rectification tower is 30, the filler in the first rectification tower is stainless steel mesh, the diameter of the tower is 600mm, and the height of the tower is 12m; collect aqueous acetone that contains about 55 wt.% acetone from the tower top, and return the aqueous acetone as raw acetone to the slurry bed reactor; while cool down the liquid of the tower bottom to room temperature, and extract for 3 times with tetrachloroethylene, combine the extract and distill

the combined extract at a reduced pressure at about 65°C to remove the tetrachloroethylene and obtain acetone oxime product; the melting point of the acetone oxime product is 58.3-60.0°C, and the Infrared Spectrum and the Mass Spectrum data of the acetone oxime product is as follows: IR, $\nu_{max}/cm^{-1}$: 3201, 2920, 2898, 1681, 1499, 1371, 1269, 1072, 949, 81; MS *m/z*(%)*:* 73 ($M^+$, 100), 58 (64), 54 (21), 42 (21), 41 (21), 31 (31), 28 (42), 15 (45).

Allow the above process to proceed for 200h continuously, and calculate the utilization ratio of the hydrogen peroxide and the selectivity of the acetone oxime at different times, according to the feeding amount and the residual amount of the acetone and the yield of the acetone oxime. The result is shown in the following Table 1.

Example 4

This example is provided to described the method for preparation of ketoxime compound intermediate provided in the method of the present invention.

Prepare acetone oxime as the method described in example 1, with the following difference: the molar ratio of hydrogen peroxide added in the form of hydrogen peroxide solution to the acetone is 0.95:1.

Allow the above process to proceed for 200h continuously, and calculate the utilization ratio of the hydrogen peroxide and the selectivity of the acetone oxime at different times, according to the feeding amount and the residual amount of the acetone and the yield of the acetone oxime. The result is shown in the following Table 1.

Example 5

This example is provided to describe the method for preparation of ketoxime compound intermediate provided in the method of the present invention.

Prepare acetone oxime as the method described in example 1, with the following difference: the molar ratio of hydrogen peroxide added in the form of hydrogen peroxide solution to the acetone is 0.5:1.

Allow the above process to proceed for 200h continuously, and calculate the utilization ratio of the hydrogen peroxide and the selectivity of the acetone oxime at different times, according to the feeding amount and the residual amount of the acetone and the yield of the acetone oxime. The result is shown in the following Table 1.

Table 1

| | | Example 1 | Comparative example 1 | Example 2 | Example 3 | Example 4 | Example 5 |
|---|---|---|---|---|---|---|---|
| 50h | Utilization ratio of the hydrogen peroxide (%) | 75.7 | 89.2 | 82.8 | 79.6 | 73.2 | 82.7 |
| | Selectivity of the acetone oxime (%) | 99.8 | 99.5 | 99.7 | 99.6 | 99.5 | 99.7 |
| 100 h | Utilization ratio of the hydrogen peroxide (%) | 74.6 | 83.1 | 82.0 | 79.8 | 72.9 | 82.4 |
| | Selectivity of the acetone oxime (%) | 99.7 | 98.2 | 99.6 | 99.6 | 99.3 | 99.6 |
| 125 h | Utilization ratio of the hydrogen peroxide (%) | 74.8 | 72.1 | 81.8 | 80.1 | 73.1 | 82.0 |
| | Selectivity of the acetone oxime (%) | 99.7 | 93.1 | 99.6 | 99.5 | 99.3 | 99.6 |
| 150 h | Utilization ratio of the hydrogen peroxide (%) | 73.8 | 43.8 | 82.1 | 79.7 | 71.6 | 81.5 |
| | Selectivity of the acetone oxime (%) | 99.6 | 72.1 | 99.6 | 99.4 | 99.0 | 99.5 |
| 175 h | Utilization ratio of the hydrogen peroxide (%) | 73.2 | - | 80.7 | 78.2 | 67.8 | 80.8 |
| | Selectivity of the acetone oxime (%) | 99.5 | - | 99.5 | 99.3 | 88.3 | 99.4 |

(continued)

| | | Example 1 | Comparative example 1 | Example 2 | Example 3 | Example 4 | Example 5 |
|---|---|---|---|---|---|---|---|
| 200 h | Utilization ratio of the hydrogen peroxide (%) | 74.3 | - | 80.9 | 79.1 | 53.4 | 80.5 |
| | Selectivity of the acetone oxime (%) | 99.5 | - | 99.5 | 99.4 | 73.2 | 99.5 |

It can be seen from the data in Table 1: with the method for preparation of acetone oxime provided in the present invention, the selectivity of the acetone oxime is high, and the useful life of the titanium-silicon molecular sieve catalyst is very long.

Example 6

This example is provided to describe the method for preparation of alkoxy-amine hydrochloride provided in the present invention.

(1) Preparation of acetone oxime

Prepare acetone oxime as the method described in example 1.

(2) Preparation of acetone oxime methyl ether

Feed 70.2g (0.96mol) acetone oxime, 220g dimethyl sulfoxide (DMSO), and 44.2g (1.1mol) NaOH into a 500ml four-neck flask equipped with an agitator at room temperature, and then feed 56g (1.11mol) monochloro methane at 25°C while agitating the solution; next, allow the materials to react for 2h at 30-40°C, and then cool down the solution to room temperature, add 220g water, extract for 3 times with 150g normal hexane, combine the extract, and distill the extract to remove the solvent, to obtain acetone oxime methyl ether; the Nuclear Magnetic Resonance Spectrum data and the Gas Chromatograph-Mass Spectrum (GC-MS) data of the obtained product is as follows:

$^1$H NMR (400 MHz, DMSO-d6) $\delta$: 1.76~1.79 (6H, d, $CH_3$), 3.71 (3H, s, $CH_3$);
MS *m*/*z* (%): 87 ($M^+$, 100), 72 (17), 56 (68).

(3) Preparation of methoxy-amine hydrochloride

Feed the acetone oxime methyl ether prepared in step (2) and hydrochloric acid solution at 25 wt.% concentration (at 320kg/h flow rate) into a second rectification tower through a first feed inlet and a second feed inlet respectively, wherein, the theoretical plate number of the second rectification tower is 80, the filler is Mellapak™ 500 (buy from Sulzer), the diameter of the tower is 400mm, the height of the tower is 35m, the theoretical plate number from the first feed inlet to the tower bottom is 32, the theoretical plate number from the second feed inlet to the tower bottom is 48, and the acetone oxime methyl ether and the hydrochloric acid solution are added in such amounts that the molar ratio of the acetone oxime methyl ether to HCl in the input materials is 1:1.5. Allow the materials in the second rectification tower to react, and separate the reaction products by rectification in the second rectification tower, by controlling the temperature at the tower bottom of the second rectification tower at about 120°C and the temperature at the tower top at about 57°C. Separate the distillate from the tower top further by rectification, to obtain aqueous acetone that contains about 55 wt.% acetone, and feed the aqueous acetone as raw acetone in step (1) into the slurry bed reactor; distill the liquid of the tower bottom at a reduced pressure, until solid is separated out; cool down the liquid to room temperature, filter, and wash with methanol, and then dry in vacuum, to obtain methoxy-amine hydrochloride product; the melting point of the methoxy-amine hydrochloride product is 150-152°C, and the Infrared Spectrum data of the methoxy-amine hydrochloride product and the Mass Spectrum data of the extract product after neutralization is as follows:

IR, $\nu_{max}/cm^{-1}$: 3014, 3003, 2986, 2860, 2739, 2713, 2620, 1578, 1507, 1403, 1189, 1146, 1036, 882,455;
MS *m/z(%):* 47 ($M^+$, 100), 32 (42), 31 (41), 29 (17), 17 (13).

According to the yield of the methoxy-amine hydrochloride and the inputted amount of the acetone oxime methyl

ether, the yield of the methoxy-amine hydrochloride is calculated as 93.0%, and the yield of the acetone is calculated as 93.2%. It can be seen: 0.051 ton external acetone is required to produce 1 ton methoxy-amine hydrochloride.

Example 7

This example is provided to describe the method for preparation of alkoxy-amine hydrochloride provided in the present invention.

Prepare methoxy-amine hydrochloride as the method described in example 6, except that: the process of the preparation of the methoxy-amine hydrochloride in step (3) is: feed the acetone oxime methyl ether prepared in step (2), hydrochloric acid solution at 25 wt.% concentration, and toluene into the tower bottom of a second rectification tower, wherein, the theoretical plate number of the second rectification tower is 80, the acetone oxime methyl ether and the hydrochloric acid solution are added in such amounts that the molar ratio of the acetone oxime methyl ether to HCl in the input materials is 1:1.5 and the weight ratio of the toluene to the acetone oxime methyl ether is 2:1. Allow the materials in the second rectification tower to react for 16h, and separate the reaction products by rectification in the second rectification tower, by controlling the temperature at the tower bottom of the second rectification tower at approx. 110°C and the temperature at the tower top at approx. 90°C. Distill the liquid of the tower bottom at a reduced pressure until solid is separated out, cool down the liquid to room temperature, filter, wash with methanol, and dry in vacuum, to obtain methoxy-amine hydrochloride product; the melting point of the methoxy-amine hydrochloride is 149-151 °C. According to the yield of the methoxy-amine hydrochloride and the inputted amount of acetone oxime methyl ether in step (3), the yield of the methoxy-amine hydrochloride is calculated as 78.5%.

Example 8

This example is provided to describe the method for preparation of alkoxy-amine hydrochloride provided in the present invention.

Prepare methoxy-amine hydrochloride as the method described in example 7, except that: no toluene is added in the process of preparation of methoxy-amine hydrochloride in step (3). According to the yield of the methoxy-amine hydrochloride and the inputted amount of the acetone oxime methyl ether in step (3), the yield of the methoxy-amine hydrochloride is calculated as 67.3%.

Example 9

This example is provided to describe the method for preparation of alkoxy-amine hydrochloride provided in the present invention.

Prepare methoxy-amine hydrochloride as the method described in example 6, except that: in the process of preparation of methoxy-amine hydrochloride in step (3), the acetone oxime methyl ether prepared in step (2) is fed into the second rectification tower through the second feed inlet, and the hydrochloric acid solution at 25 wt.% concentration is fed into the second rectification tower through the first feed inlet, that is to say, the feed inlet of the acetone oxime methyl ether is above the feed inlet of the hydrochloric acid solution.

According to the yield of the methoxy-amine hydrochloride and the inputted amount of the acetone oxime methyl ether in step (3), the yield of the methoxy-amine hydrochloride is calculated as 87.6%.

Example 10

This example is provided to describe the method for preparation of alkoxy-amine hydrochloride provided in the present invention.

Prepare methoxy-amine hydrochloride as the method described in example 6, except that: in the process of the preparation of the methoxy-amine hydrochloride in step (3), both the acetone oxime methyl ether prepared in step (2) and the hydrochloric acid solution at 25 wt.% concentration are fed into the second rectification tower through the second feed inlet, that is to say, the feed inlet of the acetone oxime methyl ether is the same as the feed inlet of the hydrochloric acid solution.

According to the yield of the methoxy-amine hydrochloride and the inputted amount of the acetone oxime methyl ether in step (3), the yield of the methoxy-amine hydrochloride is calculated as 90.4%.

Example 11

This example is provided to describe the method for preparation of alkoxy-amine hydrochloride provided in the present invention.

Prepare methoxy-amine hydrochloride as the method described in example 6, except that: in the process of the preparation of the methoxy-amine hydrochloride in step (3), the acetone oxime methyl ether is fed into the second rectification tower through the first feed inlet, and the theoretical plate number from the first feed inlet to the tower bottom is 20; the hydrochloric acid solution at 25 wt.% concentration is fed into the second rectification tower through the second feed inlet, and the theoretical plate number from the second feed inlet to the tower bottom is 72.
According to the yield of the methoxy-amine hydrochloride and the inputted amount of the acetone oxime methyl ether in step (3), the yield of the methoxy-amine hydrochloride is calculated as 83.2%.

Example 12

This example is provided to describe the method for preparation of alkoxy-amine hydrochloride provided in the present invention.

(1) Preparation of acetone oxime
Prepare acetone oxime as the method described in example 2.
(2) Preparation of acetone oxime ethyl ether
Feed 70.2g (0.96 mol) acetone oxime, 220g dimethyl sulfoxide (DMSO), and 44.2g (1.1mol) NaOH into a 500ml four-neck flask equipped with an agitator at room temperature, and then add 77.4g (1.2mol) monochloro ethane in droplets at 25°C while agitating the solution; next, allow the materials to react for 1h at 60°C, and then cool down the solution to room temperature, add 220g water, extract for 3 times with 150g normal hexane, combine the extract, and distill the extract to remove the solvent, to obtain acetone oxime ethyl ether; the Nuclear Magnetic Resonance Spectrum data and GC-MS data of the obtained product is as follows:

$^1$H NMR (400 MHz, DMSO-d6) $\delta$: 1.17 (3H, t, $CH_2CH_3$), 1.77~1.79 (6H, d, $CH_3$), 3.95~4.00 (2H, m, $CH_2$);
MS *m*/*z* (%): 101 ($M^+$, 52), 73 (100), 58 (58), 56 (66).

(3) Preparation of ethoxy-amine hydrochloride
Feed the acetone oxime ethyl ether prepared in step (2) and hydrochloric acid solution at 25 wt.% concentration (at 320kg/h flow rate) into a second rectification tower through a first feed inlet and a second feed inlet respectively, wherein, the theoretical plate number of the second rectification tower is 80, the filler is Mellapak™ 500 (buy from Sulzer), the diameter of the tower is 400mm, the height of the tower is 35m, the theoretical plate number from the first feed inlet to the tower bottom is 32, the theoretical plate number from the second feed inlet to the tower bottom is 48, and the acetone oxime ethyl ether and the hydrochloric acid solution are added in such amounts that the molar ratio of the acetone oxime ethyl ether to HCl in the input materials is 1:2. Allow the materials in the second rectification tower to react, and separate the reaction products by rectification in the second rectification tower, by controlling the temperature at the tower bottom of the second rectification tower at about 120°C and the temperature at the tower top at about 60°C. Separate the distillate from the tower top further by rectification, to obtain aqueous acetone that contains about 55 wt.% acetone, and feed the aqueous acetone as raw acetone in step (1) into the slurry bed reactor; distill the liquid of the tower bottom at a reduced pressure, until solid is separated out; cool down the liquid to room temperature, filter, and wash with methanol, and then dry in vacuum, to obtain ethoxy-amine hydrochloride product; the melting point of the ethoxy-amine hydrochloride product is 131-133°C, and the Infrared Spectrum data of the ethoxy-amine hydrochloride product and the Mass Spectrum data of the extract product after neutralization is as follows:

IR, $\nu_{max}$/cm$^{-1}$: 3426, 2987, 2701, 2280, 1978, 1612, 1403, 1153, 1102, 1034, 835, 485;
MS *m*/*z*(%): 61 ($M^+$, 19), 43 (13), 33 (100), 29 (47), 27 (28).

According to the yield of the ethoxy-amine hydrochloride and the inputted amount of the acetone oxime ethyl ether in step (3), the yield of the ethoxy-amine hydrochloride is calculated as 93.5%, and the yield of the acetone is calculated as 94.8%. It can be seen: 0.033 ton external acetone is required to produce 1 ton ethoxy-amine hydrochloride.

Example 13

This example is provided to describe the method for preparation of alkoxy-amine hydrochloride provided in the present invention.

(1) Preparation of butanone oxime

Feed butanone, hydrogen peroxide solution at 30 wt.% concentration, ammonia gas, water, and TS-1 molecular sieve catalyst (prepared by using the method disclosed in example 3 in CN1301599A) continuously into a 5L slurry bed reactor equipped with a ceramic membrane separator, the weight ratio of the amount of the butanone : the water : the TS-1 molecular sieve catalyst as 1:3:0.08, the molar ratio of the ammonia gas to the butanone is 1.7:1, and the molar ratio of hydrogen peroxide added in the form of hydrogen peroxide solution to the butanone is 0.7:1, and then allow the raw materials of reaction to react with each other for 0.5h at 80°C by controlling the input flow and output flow of the slurry bed reactor. Meanwhile, separate the reacted materials discharged from the slurry bed reactor into reacted TS-1 molecular sieve catalyst and reacted liquid via the ceramics membrane separator, and return the reacted TS-1 molecular sieve catalyst to the slurry bed reactor as raw TS-1 molecular sieve catalyst to the slurry bed reactor by back-flushing; feed the reacted liquid into the a first rectification tower for rectification and separation, wherein, the theoretical plate number of the first rectification tower is 30, the filler in the first rectification tower is stainless steel mesh, the diameter of the tower is 600mm, and the height of the tower is 12m; collect aqueous butanone that contains about 56 wt.% butanone from the tower top, and return the aqueous butanone as raw butanone to the slurry bed reactor; while cool down the liquid of the tower bottom to room temperature, and extract for 3 times with tetrachloroethylene, combine the extract and distill the combined extract at a reduced pressure at about 65 °C to remove the tetrachloroethylene and obtain butanone oxime product; the melting point of the butanone oxime product is 58.2-60.1°C, and the Infrared Spectrum and the Mass Spectrum data of the butanone oxime product is as follows:

IR, $\nu_{max}$/$cm^{-1}$: 3246, 2973, 2941, 2922, 2881, 1665, 1460, 1372, 1369, 1328, 1271, 1242, 1220, 1098, 1072, 977, 935, 800, 795, 788, 771, 611, 523;
MS *m*/*z* (%): 87 ($M^+$, 98), 86 (26), 58 (38), 42 (100), 41 (26), 29 (31), 28 (28), 27 (29);

(2) Preparation of butanone oxime methyl ether

Feed 83.6g (0.96 mol) butanone oxime, 220g dimethyl sulfoxide (DMSO), and 44.2g (1.1mol) NaOH into a 500ml four-neck flask equipped with an agitator, and then add 53g (1.05mol) monochloro methane in droplets at 25°C while agitating the solution; next, allow the materials to react for 3h at 50°C, and then cool down the solution to room temperature, add 220g water, extract for 3 times with 150g normal hexane, combine the extract, and distill the extract to remove the solvent, to obtain butanone oxime methyl ether (in cis form and trans form); the Nuclear Magnetic Resonance Spectrum data and the GC-MS data of the obtained product is as follows:

$^1$H NMR (400 MHz, DMSO-d6) *(I) $\delta$*: 1.03 (3H, t, $CH_2CH_3$), 1.77 (3H, s, $CH_3$), 2.25 (2H, m, $CH_2CH_3$), 3.72 (3H, s, $OCH_3$); (II) $\delta$: 0.98 (3H, t, $CH_2CH_3$), 1.75 (3H, s, $CH_3$), 2.14 (2H, m, $CH_2CH_3$), 3.72 (3H, s, $OCH_3$);
MS *m*/*z* (%): 101 ($M^+$, 99), 86 (17), 68 (40), 56 (23), 42 (100), 29 (27).

(3) Preparation of methoxy-amine hydrochloride

Feed the butanone oxime methyl ether prepared in step (2) and hydrochloric acid solution at 25 wt.% concentration into a second rectification tower through a first feed inlet and a second feed inlet respectively, wherein, the theoretical plate number of the second rectification tower is 80, the filler is Mellapak™ 500 (buy from Sulzer), the diameter of the tower is 400mm, the height of the tower is 35m, the theoretical plate number from the first feed inlet to the tower bottom is 32, the theoretical plate number from the second feed inlet to the tower bottom is 48, and the butanone oxime methyl ether and the hydrochloric acid solution are added in such amounts that the molar ratio of the butanone oxime methyl ether to HCl in the input materials is 1:1.8. Allow the materials in the second rectification tower to react, and separate the reaction products by rectification in the second rectification tower, by controlling the temperature at the tower bottom of the second rectification tower at about 120°C and the temperature at the tower top at about 60 °C. Separate the distillate from the tower top further by rectification, to obtain aqueous butanone that contains about 55 wt.% butanone, and feed the aqueous butanone as raw butanone in step (1) into the slurry bed reactor; distill the liquid of the tower bottom at a reduced pressure, until solid is separated out; cool down the liquid to room temperature, filter, and wash with methanol, and then dry in vacuum, to obtain methoxy-amine hydrochloride product; the melting point of the methoxy-amine hydrochloride product is 150-152°C, and the Infrared Spectrum data of the methoxy-amine hydrochloride product and the Mass Spectrum data of the extract product after neutralization is as follows:

IR, $\nu_{max}$/$cm^{-1}$: 3014, 3003, 2986, 2860, 2739, 2713, 2620, 1578, 1507, 1403, 1189, 1146, 1036, 882,455;
MS *m*/*z* (%): 47 ($M^+$, 100), 32 (42), 31 (41), 29 (17), 17 (13).

According to the yield of the methoxy-amine hydrochloride and the inputted amount of the butanone oxime methyl ether, the yield of the methoxy-amine hydrochloride is calculated as 92.5%, and the yield of the butanone is calculated as 91.3%. It can be seen: 0.081 ton external butanone is required to produce 1 ton methoxy-amine hydrochloride.

## Claims

1. A method for preparation of alkoxy-amine hydrochloride, comprising the following steps:

   (1) feeding a ketone compound, hydrogen peroxide, water, and ammonia into a reactor for contact reaction under the conditions of oximation reaction of ketone and ammonia, in the presence of a titanium-silicon molecular sieve catalyst, to prepare ketoxime compound, wherein, the molar ratio of the hydrogen peroxide to the ketone compound is 0.1 or more and less than 1;
   (2) allowing the ketoxime compound obtained in step (1) to have contact reaction with a halohydrocarbon under the conditions of substitution reaction, and separating ketoxime ether compound from the product of the contact reaction;
   (3) feeding the ketoxime ether compound obtained in step (2) and hydrochloric acid solution respectively into a second rectification tower continuously for catalytic distillation, and separating alkoxy-amine hydrochloride from the liquid at the tower bottom of the second rectification, tower, wherein the process for feeding the ketoxime ether compound obtained in step (2) and the hydrochloric acid solution respectively into the second rectification tower continuously comprises: feeding the ketoxime ether compound obtained in step (2) into the second rectification tower through a first feed inlet, and feeding the hydrochloric acid solution into the second rectification tower through a second feed inlet, wherein, the number of plates or the theoretical plate number D1 from the first feed inlet to the tower bottom and the number of plates or the theoretical plate number D2 from the second feed inlet to the tower bottom is 30-80% of the total number of plates or the theoretical plate number of the second rectification tower respectively, and D1<D2.

2. The method according to claim 1, wherein, in step (3), the total number of plates or the theoretical plate number of the second rectification tower is 20-100, preferably 30-80.

3. The method according to claim 1, wherein, in step (3), the temperature at the tower bottom of the second rectification tower is 70-110°C, preferably 85-95°C; the temperature at the tower top is 50-65°C, preferably 55-60°C.

4. The method according to claim 1, wherein, in step (3), the molar ratio of HCl in hydrochloric acid solution to the ketoxime compound in step (2) is 1.1-2.5:1, preferably 1.5-2:1; the concentration of the hydrochloric acid solution is 15-35 wt.%, preferably 20-25 wt.%.

5. The method according to claim 1, wherein, in step (2), the contact reaction between the ketoxime compound and the halohydrocarbon is performed in the presence of an alkali metal hydroxides and a solvent; preferably, the alkali metal hydroxide is sodium hydroxide and/or potassium hydroxide; the solvent is at least one of dimethyl sulfoxide, sulfolane, [BMIM]Cl ion liquid, and [BMIM]OH ion liquid; preferably, the weight ratio of the solvent to the ketoxime compound is 1-20:1, preferably 3-20:1; the molar ratio of the alkali metal hydroxide to the ketoxime compound is 1-1.5:1, preferably 1.1-1.3:1.

6. The method according to any of claim 1 or 5, wherein, in step (2), the conditions of the substitution reaction include: the reaction temperature is 20-70°C, the reaction time is 1-3h.

7. The method according to any of claim 1 or 5, wherein, in step (2), the molar ratio of the halohydrocarbon to the ketoxime compound is 1-1.3:1, preferably 1.05-1.2:1.

8. The method according to claim 1, wherein, in step (2), the general formula of the halohydrocarbon is $R_1X_t$, wherein, $R_1$ is $C_1$-$C_6$ alkyl, $C_2$-$C_6$ vinyl, $C_7$-$C_{13}$ aryl, or halogenated group of these groups, X is chlorine or bromine, t is the number of the X atom, and t is 1; the halohydrocarbon is preferably methyl chloride, ethyl chloride, 1-chloropropylene, or benzyl chloride.

9. The method according to claim 1, further comprising: in step (3), recycling and reusing the distillate from the tower top of the second rectification tower as at least a part of the ketone compound in step (1) directly or after treatment

by concentration.

**10.** The method according to claim 1, wherein, in step (1), the molar ratio of the hydrogen peroxide to the ketone compound is 0.5-0.95, preferably 0.7-0.9; the conditions of oximation reaction of ketone and ammonia include: the reaction temperature is 45-85°C, preferably 50-80°C, the reaction time is 0.5-6h, preferably 1-5h; the weight ratio of the titanium-silicon molecular sieve catalyst to the ketone compound is 0.03-0.15:1, preferably 0.05-0.1:1; the weight ratio of the water to the ketone compound is 0.5-6:1, preferably 1-4:1; the molar ratio of the ammonia to the ketone compound is 1-1.8:1, preferably 1.5-1.7:1.

**11.** The method according to claim 1, wherein, in step (1), the method further comprising:

separating the titanium-silicon molecular sieve catalyst after the contact reaction and the mixed liquid A that contains ketoxime compound, the ketone compound and water from the product of the contact reaction, separating the ketoxime compound and the mixed liquid B that contains the ketone compound and water from the mixed liquid A, and then feeding the mixed liquid B as a part of input materials of the reactor back to the reactor.

**12.** The method according to claim 11, wherein, the process for separating the ketoxime compound and the mixed liquid B that contains the ketone compound and water from the mixed liquid A is performed in a first rectification tower, and wherein, the theoretical plate number of the first rectification tower is 20-80, preferably 30-60; the temperature at the tower bottom is 80-130°C, preferably 100-120°C; and the reflux ratio is not lower than 2:1, preferably 5-10:1.

**13.** The method according to claim 11, wherein, in step (1), the method further comprising:

recycling and reusing the separated titanium-silicon molecular sieve catalyst after the contact reaction as at least a part of the titanium-silicon molecular sieve catalyst for the contact reaction.

**14.** The method according to claim 11, wherein, in step (1), the process for separating of the titanium-silicon molecular sieve catalyst after the contact reaction and the mixed liquid A from the product of the contact reaction is implemented in a membrane filter.

**15.** The method according to any of claims 1 and 10-14, wherein, in step (1), the ketone compound is $C_3$-$C_6$ monoketone, preferably acetone or butanone; the titanium-silicon molecular sieve catalyst is a titanium-silicon molecular sieve catalyst with a hollow structure.

## Patentansprüche

**1.** Verfahren zur Herstellung von Alkoxy-Amin-Hydrochlorid, umfassend die folgenden Schritte:

(1) Einspeisen von einer Ketonverbindung, Wasserstoffperoxid, Wasser und Ammoniak in einen Reaktor für Kontaktreaktion unter den Bedingungen von Oximierungsreaktion von Keton und Ammoniak in Gegenwart eines Titan-Silicium-Molekularsiebkatalysators, um eine Ketoximverbindung herzustellen, wobei das Molverhältnis vom Wasserstoffperoxid zur Ketonverbindung 0,1 oder höher und niedriger als 1 ist;
(2) Zulassen, dass die in Schritt (1) erhaltene Ketoximverbindung unter den Bedingungen von Substitutionsreaktion mit einem Halokohlenwasserstoff Kontakt hat, und Trennen der Ketoximetherverbindung vom Produkt der Kontaktreaktion;
(3) jeweiliges kontinuierliches Einspeisen der in Schritt (2) erhaltenen Ketoximetherverbindung und einer Chlorwasserstoffsäurelösung in eine zweite Rektifikationssäule für katalytische Destillation und Trennen von Alkoxy-Amin-Hydrochlorid von der Flüssigkeit am unteren Säulenende der zweiten Rektifikationssäule, wobei der Prozess zum jeweiligen kontinuierlichen Einspeisen der in Schritt (2) erhaltenen Ketoximetherverbindung und der Chlorwasserstoffsäurelösung in die zweite Rektifikationssäule umfasst: Einspeisen der in Schritt (2) erhaltenen Ketoximetherverbindung in die zweite Rektifikationssäule durch einen ersten Beschickungseinlass, und Einspeisen der Chlorwasserstoffsäurelösung in die zweite Rektifikationssäule durch einen zweiten Beschickungseinlass, wobei die Anzahl von Platten oder die theoretische Plattenanzahl D1 vom ersten Beschickungseinlass zum unteren Säulenende und die Anzahl von Platten oder die theoretische Plattenanzahl D2 vom zweiten Beschickungseinlass zum unteren Säulenende 30 bis 80 % der Gesamtanzahl von Platten bzw. der theoretischen Plattenanzahl der zweiten Rektifikationssäule beträgt, und D1 < D2.

2. Verfahren nach Anspruch 1, wobei in Schritt (3) die Gesamtanzahl von Platten oder die theoretische Plattenanzahl der zweiten Rektifikationssäule 20 bis 100 und vorzugsweise 30 bis 80 beträgt.

3. Verfahren nach Anspruch 1, wobei in Schritt (3) die Temperatur am unteren Säulenende der zweiten Rektifikationssäule 70 bis 110 °C und vorzugsweise 85 bis 95 °C beträgt; und die Temperatur am oberen Säulenende 50 bis 65 °C und vorzugsweise 55 bis 60 °C beträgt.

4. Verfahren nach Anspruch 1, wobei in Schritt (3) das Molverhältnis von HCl in der Chlorwasserstoffsäurelösung zur Ketoximverbindung in Schritt (2) 1,1 bis 2,5:1 und vorzugsweise 1,5 bis 2:1 beträgt; und die Konzentration der Chlorwasserstoffsäurelösung 15 bis 35 Gew.-% und vorzugsweise 20 bis 25 Gew.-% beträgt.

5. Verfahren nach Anspruch 1, wobei in Schritt (2) die Kontaktreaktion zwischen der Ketoximverbindung und dem Halokohlenwasserstoff in Gegenwart von Alkalimetallhydroxiden und eines Lösungsmittels durchgeführt wird; das Alkalimetallhydroxid vorzugsweise Natriumhydroxid und/oder Kaliumhydroxid ist; das Lösungsmittel mindestens eines von Dimethylsulfoxid, Sulfolan, [BMIM]Cl-Ionenflüssigkeit und [BMIM]OH-Ionenflüssigkeit ist; vorzugsweise das Gewichtsverhältnis vom Lösungsmittel zur Ketoximverbindung 1 bis 20:1 und vorzugsweise 3 bis 20:1 ist; das Molverhältnis vom Alkalimetallhydroxid zur Ketoximverbindung 1 bis 1,5:1 und vorzugsweise 1,1 bis 1,3:1 ist.

6. Verfahren nach einem der Ansprüche 1 oder 5, wobei in Schritt (2) die Bedingungen der Substitutionsreaktion umfassen, dass die Reaktionstemperatur 20 bis 70 °C beträgt, und die Reaktionszeit 1 bis 3 h beträgt.

7. Verfahren nach einem der Ansprüche 1 oder 5, wobei in Schritt (2) das Molverhältnis vom Halokohlenwasserstoff zur Ketoximverbindung 1 bis 1,3:1 und vorzugsweise 1,05 bis 1,2:1 beträgt.

8. Verfahren nach Anspruch 1, wobei in Schritt (2) die allgemeine Formel des Halokohlenwasserstoffs $R_1X_t$ ist, wobei $R_1$ $C_1$-$C_6$-Alkyl, $C_2$-$C_6$-Vinyl, $C_7$-$C_{13}$-Aryl oder eine halogenierte Gruppe dieser Gruppen ist, X Chlor oder Brom ist, t die Anzahl des X-Atoms ist, und t 1 ist; und der Halokohlenwasserstoff vorzugsweise Methylchlorid, Ethylchlorid, 1-Chlorpropylen oder Benzylchlorid ist.

9. Verfahren nach Anspruch 1, ferner umfassend in Schritt (3) ein Rezirkulieren und Wiederverwenden des Destillats aus dem oberen Säulenende der zweiten Rektifikationssäule als wenigstens einen Teil der Ketonverbindung in Schritt (1) entweder direkt oder nach einer Behandlung durch Konzentration.

10. Verfahren nach Anspruch 1, wobei in Schritt (1) das Molverhältnis vom Wasserstoffperoxid zur Ketonverbindung 0,5 bis 0,95 und vorzugsweise 0,7 bis 0,9 beträgt; die Bedingungen der Oximierungsreaktion von Keton und Ammoniak umfassen, dass die Reaktionstemperatur 45 bis 85 °C und vorzugsweise 50 bis 80 °C beträgt, die Reaktionszeit 0,5 bis 6 h und vorzugsweise 1 bis 5 h beträgt; das Gewichtsverhältnis vom Titan-Silicium-Molekularsiebkatalysator zur Ketonverbindung 0,03 bis 0,15:1 und vorzugsweise 0,05 bis 0,1:1 beträgt; das Gewichtsverhältnis vom Wasser zur Ketonverbindung 0,5 bis 6:1 und vorzugsweise 1 bis 4:1 beträgt; und das Molverhältnis vom Ammoniak zur Ketonverbindung 1 bis 1,8:1 und vorzugsweise 1,5 bis 1,7:1 beträgt.

11. Verfahren nach Anspruch 1, wobei in Schritt (1) das Verfahren ferner umfasst: Trennen des Titan-Silicium-Molekularsiebkatalysators nach der Kontaktreaktion und der gemischten Flüssigkeit A, welche die Ketoximverbindung, die Ketonverbindung und Wasser enthält, vom Produkt der Kontaktreaktion, Trennen der Ketoximverbindung und der gemischten Flüssigkeit B, welche die Ketonverbindung und Wasser enthält, von der gemischten Flüssigkeit A und anschließendes Einspeisen der gemischten Flüssigkeit B als einen Teil von Eingabematerialien des Reaktors zurück in den Reaktor.

12. Verfahren nach Anspruch 11, wobei der Prozess zum Trennen der Ketoximverbindung und der gemischten Flüssigkeit B, welche die Ketonverbindung und Wasser enthält, von der gemischten Flüssigkeit A in einer ersten Rektifikationssäule erfolgt, und wobei die theoretische Plattenanzahl der ersten Rektifikationssäule 20 bis 80 und vorzugsweise 30 bis 60 beträgt; die Temperatur am unteren Säulenende 80 bis 130 °C und vorzugsweise 100 bis 120 °C beträgt; und das Rückflussverhältnis nicht niedriger als 2:1 und vorzugsweise 5 bis 10:1 ist.

13. Verfahren nach Anspruch 11, wobei in Schritt (1) das Verfahren ferner umfasst: Rezirkulieren und Wiederverwenden des getrennten Titan-Silicium-Molekularsiebkatalysators nach der Kontaktreaktion als mindestens einen Teil des Titan-Silicium-Molekularsiebkatalysators für die Kontaktreaktion.

**14.** Verfahren nach Anspruch 11, wobei in Schritt (1) der Prozess zum Trennen des Titan-Silicium-Molekularsiebkatalysators nach der Kontaktreaktion und der gemischten Flüssigkeit A vom Produkt der Kontaktreaktion in einem Membranfilter implementiert wird.

**15.** Verfahren nach einem der Ansprüche 1 und 10 bis 14, wobei in Schritt (1) die Ketonverbindung $C_3$-$C_6$-Monoketon, vorzugsweise Aceton oder Butanon, ist; und der Titan-Silicium-Molekularsiebkatalysator ein Titan-Silicium-Molekularsiebkatalysator mit einer hohlen Struktur ist.

## Revendications

**1.** Procédé de préparation d'un chlorhydrate d'alcoxyamine, comprenant les étapes suivantes consistant à :

(1) alimenter un composé cétone, du peroxyde d'hydrogène, de l'eau et de l'ammoniac dans un réacteur pour une réaction de contact dans les conditions de réaction d'oximation de la cétone et de l'ammoniac, en présence d'un catalyseur de tamis moléculaire de titane-silicium, afin de préparer un composé cétoxime, dans lequel le rapport molaire du peroxyde d'hydrogène au composé cétone est supérieur ou égal à 0,1 et inférieur à 1 ;
(2) laisser le composé cétoxime obtenu dans l'étape (1) avoir une réaction de contact avec un halogénohydrocarbure dans les conditions de réaction de substitution, et séparer le composé éther de cétoxime du produit de la réaction de contact ;
(3) alimenter le composé éther de cétoxime obtenu dans l'étape (2) et une solution d'acide chlorhydrique respectivement dans une seconde tour de rectification de manière continue pour une distillation catalytique, et séparer le chlorhydrate d'alcoxyamine du liquide au niveau du fond de la tour de la seconde tour de rectification, le procédé pour l'alimentation du composé éther de cétoxime obtenu dans l'étape (2) et de la solution d'acide chlorhydrique respectivement dans la second tour de rectification de manière continue consistant à : alimenter le composé éther de cétoxime obtenu dans l'étape (2) dans la seconde tour de rectification par le biais d'une première entrée d'alimentation, et alimenter la solution d'acide chlorhydrique dans la seconde tour de rectification par le biais d'une seconde entrée d'alimentation, le nombre de plaques ou le nombre de plaques théorique D1 de la première entrée d'alimentation au fond de la tour et le nombre de plaques ou le nombre de plaques théorique D2 de la seconde entrée d'alimentation au fond de la tour étant de 30 à 80 % du nombre total de plaques ou du nombre de plaques théorique de la seconde tour de rectification respectivement et D1 < D2.

**2.** Procédé selon la revendication 1, dans lequel, dans l'étape (3), le nombre total de plaques ou le nombre de plaques théorique de la seconde tour de rectification est de 20 à 100, de préférence de 30 à 80.

**3.** Procédé selon la revendication 1, dans lequel, dans l'étape (3), la température au fond de la tour de la seconde tour de rectification est de 70 à 110 °C, de préférence de 85 à 95 °C ; la température en haut de la tour est de 50 à 65 °C, de préférence de 55 à 60 °C.

**4.** Procédé selon la revendication 1, dans lequel, dans l'étape (3), le rapport molaire de l'HCl dans la solution d'acide chlorhydrique au composé cétoxime dans l'étape (2) est de 1,1 à 2,5:1, de préférence de 1,5 à 2:1 ; la concentration de la solution d'acide chlorhydrique est de 15 à 35 % en poids, de préférence de 20 à 25 % en poids.

**5.** Procédé selon la revendication 1, dans lequel, dans l'étape (2), la réaction de contact entre le composé cétoxime et l'halogénohydrocarbure est réalisée en présence d'un hydroxyde de métal alcalin et d'un solvant ; de préférence, l'hydroxyde de métal alcalin est l'hydroxyde de sodium et/ou l'hydroxyde de potassium ; le solvant est au moins un solvant choisi parmi le diméthylsulfoxyde, le sulfolane, le liquide ionique [BMIM]Cl et le liquide ionique [BMIM]OH ; de préférence, le rapport en poids du solvant au composé cétoxime est de 1 à 20:1, de préférence de 3 à 20:1 ; le rapport molaire de l'hydroxyde de métal alcalin au composé cétoxime est de 1 à 1,5:1, de préférence de 1,1 à 1,3:1.

**6.** Procédé selon l'une quelconque des revendications 1 ou 5, dans lequel, dans l'étape (2), les conditions de la réaction de substitution comprennent : la température de réaction qui est de 20 à 70 °C, le temps de réaction qui est de 1 à 3 h.

**7.** Procédé selon l'une quelconque des revendications 1 ou 5, dans lequel, dans l'étape (2), le rapport molaire de l'halogénohydrocarbure au composé cétoxime est de 1 à 1,3:1, de préférence de 1,05 à 1,2:1.

**8.** Procédé selon la revendication 1, dans lequel, dans l'étape (2), la formule générale de l'halogénohydrocarbure est $R_1X_t$ où $R_1$ est un groupe alkyle en $C_1$ à $C_6$, vinyle en $C_2$ à $C_6$, aryle en $C_7$ à $C_{13}$ ou un groupe halogéné de ces

groupes, X est un atome de chlore ou de brome, t est le nombre de l'atome X et t vaut 1 ; l'halogénohydrocarbure est de préférence le chlorure de méthyle, le chlorure d'éthyle, le 1-chloropropylène ou le chlorure de benzyle.

**9.** Procédé selon la revendication 1, comprenant en outre, dans l'étape (3), le recyclage et la réutilisation du distillat provenant du haut de la tour de la second tour de rectification sous forme d'au moins une partie du composé cétone dans l'étape (1) directement ou après le traitement par concentration.

**10.** Procédé selon la revendication 1, dans lequel, dans l'étape (1), le rapport molaire du peroxyde d'hydrogène au composé cétone est de 0,5 à 0,95, de préférence de 0,7 à 0,9 ; les conditions de la réaction d'oximation de la cétone et de l'ammoniac comprennent : la température de réaction qui est de 45 à 85 °C, de préférence de 50 à 80 °C ; le temps de réaction qui est de 0,5 à 6 h, de préférence de 1 à 5 h ; le rapport en poids du catalyseur de tamis moléculaire de titane-silicium au composé cétone qui est de 0,03 à 0,15:1, de préférence de 0,05 à 0,1:1 ; le rapport molaire de l'eau au composé cétone qui est de 0,5 à 6:1, de préférence de 1 à 4:1 ; le rapport molaire de l'ammoniac au composé cétone qui est de 1 à 1,8:1, de préférence de 1,5 à 1,7:1.

**11.** Procédé selon la revendication 1, dans lequel, dans l'étape (1), le procédé consiste en outre à :

séparer le catalyseur de tamis moléculaire de titane-silicium après la réaction de contact et le liquide A mixte qui contient le composé cétoxime, le composé cétone et l'eau du produit de la réaction de contact, séparer le composé cétoxime et le liquide B mixte qui contient le composé cétone et l'eau du liquide A mixte, et ensuite alimenter le liquide B mixte sous forme de substances d'entrée du réacteur de nouveau vers le réacteur.

**12.** Procédé selon la revendication 11, dans lequel le procédé de séparation du composé cétoxime et du liquide B mixte qui contient le composé cétone et l'eau du liquide A mixte est réalisé dans une première tour de rectification, et dans lequel, le nombre de plaques théorique de la première tour de rectification est de 20 à 80, de préférence de 30 à 60 ; la température au fond de la tour est de 80 à 130 °C, de préférence de 100 à 120 °C ; et rapport de reflux n'est pas inférieur à 2:1, de préférence 5 à 10:1.

**13.** Procédé selon la revendication 11, dans lequel, dans l'étape (1), le procédé consiste en outre à :

recycler et réutiliser le catalyseur de tamis moléculaire de titane-silicium séparé après la réaction de contact sous forme d'au moins une partie du catalyseur de tamis moléculaire de titane-silicium pour la réaction de contact.

**14.** Procédé selon la revendication 11, dans lequel, dans l'étape (1), le procédé pour la séparation du catalyseur de tamis moléculaire de titane-silicium après la réaction de contact et du liquide A mixte du produit de la réaction de contact est réalisé dans un filtre membranaire.

**15.** Procédé selon l'une quelconque des revendications 1 et 10 à 14, dans lequel, dans l'étape (1), le composé cétone est une monocétone en $C_3$ à $C_6$, de préférence l'acétone ou la butanone ; le catalyseur de tamis moléculaire de titane-silicium est un catalyseur de tamis moléculaire de titane-silicium ayant une structure creuse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1556096 A
- US 4981996 A
- CN 101503373
- EP 1548000 A
- WO 0055121 A
- CN 1301599 A


### Non-patent literature cited in the description


- **JI YONGXIN.** *Chemistry and Adhesion,* 2001, vol. 5, 200-202
- **LI WENXIAO et al.** *Fine Chemical Industrial Raw Materials & Intermediates,* 2007, vol. 22-23, 29
- **ZHOU SHAODONG et al.** *JOURNAL OF ZHEJIANG UNIVERSITY (ENGINEERING SCIENCE),* 2010, vol. 44 (6), 1169-1172
- **LIU YUEMING.** Highly Selective Synthesis of Methyl Ethyl Ketone Oxime through Ammoximation over Ti-MWW. *Applied Catalysis A: General,* 2007, vol. 327, 22-31